# EUROPÄISCHE PATENTSCHRIFT

(11) **EP 3 015 749 B1**
(45) Veröffentlichungstag und Bekanntmachung des Hinweises auf die Patenterteilung: **27.05.2020**
(21) Anmeldenummer: 14190865.7
(22) Anmeldetag: 29.10.2014
(51) Int. Cl.: F16K 7/04, A61M 5/168, A61M 39/28, F16K 31/10, A61M 5/14, F16K 11/02, F16K 25/00, F16K 27/02, F16K 31/06

(54) **Vorrichtung zur Steuerung fluidischer Medien sowie Verwendung eines Formteils in einer solchen Vorrichtung**
Device for controlling fluid media and use of such a moulded part in such a device
Dispositif de commande de milieux fluidiques et utilisation d'un élément de formage dans un tel dispositif

(43) Veröffentlichungstag der Anmeldung: 04.05.2016
(73) Patentinhaber: Asco Numatics GmbH, 75248 Ölbronn-Dürrn (DE)
(72) Erfinder: Ams, Felix, 75236 Kämpfelbach (DE); SPITTELMEISTER; Uwe, 76275 Ettlingen (DE)
(74) Vertreter: Witte, Weller & Partner Patentanwälte mbB

(56) Entgegenhaltungen:
- EP-A1- 2 314 344
- EP-A1- 2 392 842
- WO-A1-2009/149137
- DE-A1-102010 010 827
- DE-A1-102010 017 216
- DE-B1- 1 648 129
- US-A1- 2008 006 336
- US-A1- 2013 119 284
- ANDREAS LOTH ET AL: "Investigation of Different Liquid Silicone Rubbers for Micro Pinch Valves", KEY ENGINEERING MATERIALS, TRANS TECH PUBL, AEDERMANNSDORF, vol. 611-612, 1 May 2014 (2014-05-01), pages 876-882, XP009508892, ISSN: 0252-1059, DOI: 10.4028/WWW.SCIENTIFIC.NET/KEM.611-612.876

## Beschreibung

Die vorliegende Erfindung betrifft eine Vorrichtung zur Steuerung fluidischer Medien, insbesondere ein Wegeventil, das zumindest einen Strömungspfad umfasst, der sich zwischen zumindest zwei Anschlüssen erstreckt, wobei eine mit einem Aktor versehene Steuerungseinheit vorgesehen ist, wobei der Aktor mit zumindest einem Sperrglied koppelbar ist, das in einer Sperrstellung den zumindest einen Strömungspfad sperrt. Ferner betrifft die vorliegende Erfindung die Verwendung eines Formteils in einer solchen Vorrichtung, insbesondere in einem Wegeventil.

Vorrichtungen der vorgenannten Art, insbesondere Wegeventile, sind im Hause der Anmelderin bekannt. Beispielhaft sind kompakt bauende Magnetventile bekannt, die für den Einsatz im Umfeld der Medizintechnik bzw. der Analysentechnik ausgebildet sind. Derartige Ventile sollen grundsätzlich zur Steuerung aggressiver oder gar hochaggressiver Medien geeignet sein. Hierzu können auch hochreaktive Medien zählen. Gleichwohl versteht sich, dass auch eine Eignung für eine hohe Reinheit erfordernde oder allgemein empfindliche Medien gegeben sein soll.

Oft ist es in diesem Zusammenhang bevorzugt, eine bauliche Trennung zwischen Strömungspfaden, die zur Leitung der Medien ausgebildet sind, und beweglichen Bauteilen der Ventile vorzusehen, die auf die Strömungspfade einwirken können, um einen Weg für die Medien selektiv zu sperren oder freizugeben. Ein Ansatz hierfür kann darin bestehen, eine sogenannte Trennmembran vorzusehen, die zwischen einem Aktor des Ventils und zumindest einem Strömungspfad angeordnet ist und vorzugsweise zumindest einen Teil eines Leitungsabschnitts ausbildet. Eine solche Trennmembran kann demnach zumindest abschnittsweise eine hermetische (stoffliche) Trennung zwischen den strömenden Medien und dem Aktor bewirken.

Es hat sich jedoch gezeigt, dass auch bei Ventilen, die gemäß der vorbeschriebenen Art mit Trennmembranen versehen sind, Ablagerungen, Korrosion oder gar Bauteilbeschädigungen auftreten können, die auf die benutzten Medien zurückgeführt werden können. Dies kann einen erhöhten Reinigungsaufwand, Wartungsaufwand und gegebenenfalls sogar Reparaturaufwand bedingen. Umgekehrt kann auch eine Kontamination der Medien drohen, so dass ein beträchtlicher Aufwand für die Reinigung, Desinfektion oder gar Sterilisation erforderlich sein kann.

Ferner hat sich gezeigt, dass insbesondere bei kompakt bauenden Ventilen, die etwa als sogenannte Mikro-Ventile bezeichnet werden können, die erforderlichen Reinigungs-, Wartungs- und/oder Reparaturarbeiten aufgrund der erschwerten Zugänglichkeit mit Schwierigkeiten verbunden sein können. Das Bestreben nach immer kompakter bauenden Ventilen im medizinischen Umfeld, im Laborumfeld bzw. Analyseumfeld würde diese Nachteile weiter vergrößern.

Ventile, die auch für aggressive oder gar hochaggressive Medien geeignet sind, erfordern häufig zumindest bei ihren strömungsführenden Teilen die Verwendung hochwertiger Werkstoffe, etwa von Edelstahl oder von Spezialkunststoffen, die eine zumindest hinreichende Medienbeständigkeit aufweisen.

Aus der US 2008/0006336 A1 ist ein Miniaturventil bekannt, umfassend einem Ventilkörper und einem darauf positionierten Aktor, wobei der Ventilkörper eine Stützvorrichtung; einen Ventilsitz, der mit einer ersten Trennwand versehen ist und einen ersten Kanal und einen zweiten Kanal definiert, wobei der erste Kanal und der zweite Kanal durch die erste Trennwand beabstandet sind, und wobei die Stützvorrichtung und der Ventilsitz verbunden sind und gemeinsam eine Kammer definieren; eine elastische Ventilmembran in der Kammer, die die Kammer in eine Flüssigkeitskammer und eine Installationskammer unterteilt, wobei die Ventilmembran die beiden Kanäle und die erste Trennwand abdeckt; und eine Wippe aufweist, die drehbar in der Kammer angeordnet ist und in einer ersten Stellung und in einer zweiten Stellung jeweils einen der beiden Kanäle sperrt und den anderen freigibt.

Aus dem Artikel Loth et al: "Investigation of Different Liquid Silicone Rubbers for Micro Pinch Valves" (KEY ENGINEERING MATERIALS, TRANS TECH PUBL, AEDERMANNSDORF, Bd. 611-612, 1. Mai 2014 (2014-05-01), Seiten 876-882, XP009508892, ISSN: 0252-1059, DOI: 10.4028/WWW.SCIENTIFIC.NET/KEM. 611-612.876) ist ein Quetschventil für mikrofluidische Anwendungen bekannt, mit einem Formteil aus Silikon-Elastomeren, wobei das Formteil Fluidkanäle bildet.

Aus der DE 1 648 129 B1 ist eine Dosiervorrichtung zur Abgabe von Flüssigkeiten aus einer unter Druck stehenden Quelle an eine Verbrauchsstelle bekannt, bei der eine in ihrem Volumen unterschiedlich einstellbare Messkammer über eine Zuflussleitung eine Flüssigkeitsmenge aus der unter Druck stehenden Quelle gegen die Wirkung einer Federkraft aufnimmt und bei Fortfall des Druckes mittels der Federkraft über eine Abflussleitung an die Verbrauchsstelle abgibt, wobei der Messkammer je eine in der Zuflussleitung und in der Abflussleitung liegende rohrartige, elastische Manschette zugeordnet ist, die einseitig je an einem feststehenden Nocken anliegt, wobei in Gegenüberlage zu den feststehenden Nocken getrennt bewegliche, der Öffnung und der Schließung der Manschetten dienende Gegennocken vorgesehen sind, denen ein hydraulischer Druckzylinder zugeordnet ist, dessen Kolben die Gegennocken über durch Federn vorgespannte Zwischenglieder derart steuert, dass bei nicht beaufschlagtem Kolben die Zuflussmanschette offen und die Abflussmanschette geschlossen ist, während der Beaufschlagung des Kolbens zunächst die Zuflussmanschette geschlossen und darauf die Abflussmanschette geöffnet wird und bei Entlastung des Kolbens zunächst die Abflussmanschette geschlossen und dann die Zuflussmanschette geöffnet wird.

Aus der DE 10 2010 010 827 A1 ist ein Biegewandlerventil bekannt, mit einem Ventilgehäuse, in dem ein als Hohlkörper ausgebildeter Ventilkörper angeordnet ist, dessen Wandung zumindest partiell gummielastisch verformbar ist und einen durch Verformung der Wandung beweglichen Dichtabschnitt definiert, der gemeinsam mit einem gegenüberliegenden Ventilsitz eine zwischen zwei jeweils mit einer Anschlussöffnung kommunizierenden Kammerabschnitten einer Ventilkammer angeordnete Überströmöffnung begrenzt, und mit einem in dem Ventilgehäuse anordneten Biegewandler, unter dessen Mitwirkung der Dichtabschnitt wahlweise in einer an dem Ventilsitz anliegenden Schließstellung oder in mindestens einer von dem Ventilsitz abgehobenen Offenstellung positionierbar ist.

Aus der WO 2009/149137 A1 ist ein Quetschventil bekannt, mit einem flexiblen Rohrelement, einem Rohrgehäusekörper zum Halten des flexiblen Rohrelements, einem Klemmelement in direktem Kontakt mit dem flexiblen Rohrelement, und mit einer Betätigungsanordnung mit einem piezoelektrischen Element und einer Verschiebungsverstärkungsstruktur, wobei das Klemmelement mit der Verschiebungsverstärkungsstruktur der Betätigungsanordnung verbunden und gegen das flexible Rohrelement beweglich ist, um die Querschnittsfläche des flexiblen Röhrenelements zu variieren, indem das flexible Rohrelement gegen den Rohrgehäusekörper geklemmt wird.

Aus der US 2013/119284 A1 ist Quetschventil bekannt, mit einem Körper, einem elastischen Kanal, der in dem Körper gehalten wird und durch welchen ein Fluid fließt, einem Elektromagneten, der mit dem Körper verbunden ist und eine Spule aufweist, wobei der Elektromagnet durch Einschalten der Spule erregt wird, um einen beweglichen Eisenkern zu einem festen Eisenkern zu ziehen, einem Ventilmechanismus mit einem Ventilstopfen, der so angeordnet ist, dass er dem Kanal zugewandt ist, und der in einer axialen Richtung des Körpers verschiebbar ist, wobei der Ventilmechanismus den Ventilstopfen durch Erregen des Elektromagneten zu dem Kanal presst, und mit einem elastischen Mittel, welches den beweglichen Eisenkern zu dem festen Eisenkern drückt, wobei durch Verschieben des Ventilstopfens zu dem Kanal der Kanal zwischen dem Ventilstopfen und dem Körper geklemmt wird, um dadurch die Strömung des Fluides zu unterbrechen.

Aus der EP 2 314 344 A1 ist eine elektrisch betätigbare Klemme zum Abklemmen von Schläuchen bekannt, mit einem elektrischen Antrieb, zwei Scherenhebeln, die gegeneinander um eine Schwenkachse zwischen einer Öffnungsstellung und einer Klemmstellung verschwenkbar sind, und die jeweilige Klemmflächen aufweisen, und mit einer Umsetzeinrichtung, mittels welcher eine Bewegung des elektrischen Antriebs in eine Bewegung der Scherenhebel umsetzbar ist.

Vor diesem Hintergrund liegt der Erfindung die Aufgabe zugrunde, eine Vorrichtung zur Steuerung fluidischer Medien, insbesondere ein Wegeventil, anzugeben, die kompakt gestaltet sein kann, einen möglichst geringen Bauraumbedarf aufweist und sich insbesondere zur Anwendung im Bereich der Analysentechnik und/oder der Medizintechnik eignet. Die Vorrichtung soll möglichst zur Steuerung und/oder Regelung aggressiver oder gar hochaggressiver Medien geeignet sein. Ferner ist es bevorzugt, wenn die Vorrichtung in einfacher Weise gereinigt bzw. gewartet werden kann und darüber hinaus möglichst reparaturfreundlich gestaltet ist. Schließlich ist es bevorzugt, wenn die Vorrichtung kostengünstig herstellbar ist, wobei vorzugsweise endkonturnahe Fertigungsverfahren nutzbar sind, die den Fertigungsaufwand bzw. einen Bearbeitungsaufwand reduzieren können. Ferner soll im Rahmen der vorliegenden Offenbarung eine vorteilhafte Verwendung eines Formteils bei einer Vorrichtung zur Steuerung fluidischer Medien, insbesondere bei einem Wegeventil, angegeben werden.

Die Vorrichtung betreffend wird die Aufgabe der Erfindung durch eine Vorrichtung zur Steuerung fluidischer Medien nach Anspruch 1 gelöst.

Die Aufgabe der Erfindung wird auf diese Weise vollkommen gelöst.

Erfindungsgemäß erlaubt nämlich das aus dem elastomeren Werkstoff gebildete Formteil eine vollständige Trennung der genutzten Fluide von übrigen Komponenten der Vorrichtung. Somit ist die Vorrichtung insbesondere zur Steuerung und/oder Regelung aggressiver, reaktiver, empfindlicher bzw. eine hohe Reinheit erfordernder Medien, insbesondere Fluide, geeignet, da diese schlichtweg nicht über das Formteil hinaus mit anderen Komponenten in Kontakt kommen können. Mit anderen Worten kann eine vollständige Isolation bzw. hermetische Trennung zwischen den Medien und weiteren Teilen der Vorrichtung bewirkt werden. Sämtliche Strömungspfade, die die Vorrichtung aufweist, können sich vollständig entlang von Leitungsabschnitten erstrecken, die vom Formteil bereitgestellt werden.

Die Vorrichtung kann äußerst kompakt gestaltet sein. Die hermetische Trennung zwischen den zu steuernden Medien und übrigen Komponenten erlaubt eine in Bezug auf die Abmessungen der Vorrichtung optimale Auslegung der Komponenten, insbesondere der Steuereinheit sowie des Aktors. Bei der Auslegung dieser Komponenten muss nämlich nicht in besonderer Weise Rücksicht auf die Eigenschaften der Medien Rücksicht genommen werden. Mit anderen Worten kann die Vorrichtung unter Nutzung kostengünstiger Werkstoffe und einfacher Bauteilgeometrien erzeugt werden, wodurch sich Kostenvorteile bei der Herstellung und insbesondere Bauraumvorteile ergeben können. Ferner lässt sich ein etwaiger Aufwand für die Reinigung, Desinfektion oder gar Sterilisation strömungsführender Teile der Vorrichtung beträchtlich minimieren.

Das Formteil kann zumindest einen durchgehenden bzw. kontinuierlich ausgebildeten Strömungspfad bereitstellen und diesen vollständig im elastomeren Werkstoff ausbilden. Dies ist vorteilhaft gegenüber der Verwendung sogenannter Trennmembranen, da keinerlei Kontakt der Medien mit anderen Komponenten zu befürchten ist. Bei der Verwendung von Trennmembranen werden zumindest Teilabschnitte der Strömungspfade durch andere Komponenten gebildet und müssen demgemäß für die genutzten Medien geeignet sein. Dies bedingt die Verwendung teurer Werkstoffe, etwa von Edelstahl oder Ähnlichem. Dies führt etwa bei Wegeventilen für aggressive, empfindliche bzw. hochreine Medien, die auf der Verwendung von Trennmembranen beruhen, zu einem erhöhten Aufwand für die Materialbereitstellung sowie die Fertigung.

Die Verwendung zumindest eines Formteils, das zumindest einen durchgehenden Strömungspfad bereitstellt, hat den weiteren Vorteil, dass im Verschleißfalle sowie im Falle etwaiger Beschädigungen in einfacher Weise ein Austausch des Formteils erfolgen kann. Somit kann das Formteil als Austauschteil bzw. Verschleißteil betrachtet werden. Die Vorrichtung kann in einfacher Weise für einen einfachen Wechsel des Formteils ausgebildet sein. Auf diese Weise kann die Vorrichtung insbesondere auch zur Verwendung mit verschiedenartigen Medien geeignet sein, wenn in entsprechender Weise Formteile bereitgehalten werden, die dem jeweiligen Medium zugeordnet sind. Da die verschiedenen Medien jeweils nur das entsprechende Formteil kontaktieren würden, muss beim Wechsel zwischen verschiedenen Medien nicht in besonderer Weise eine Reinigung, Sterilisation oder sonstige Ertüchtigung vorgenommen werden.

Bei dem elastomeren Werkstoff kann es sich insbesondere um Silikon bzw. einen silikonbasierten Werkstoff handeln. Ferner ist die Verwendung von Fluorkunststoffen denkbar, etwa von Polytetrafluorethylen (PTFE). Allgemein ist es bevorzugt, wenn Werkstoffe verwendet werden, die eine gute thermische Beständigkeit sowie vorzugsweise eine gute chemische Beständigkeit aufweisen. Es versteht sich, dass neben den genannten Werkstoffen weitere Werkstoffe denkbar sind, die sich hinreichend elastisch verhalten und im Hinblick auf die genutzten Medien eine zumindest hinreichende Beständigkeit aufweisen. Alternative Werkstoffe für das Formteil können etwa Fluorkautschuk (FKM bzw. KPM), Perfluorkautschuk (FFKM bzw. FFPM), Ethylen-Propylen-DienKautschuk (EPDM) und ähnliche elastomere Werkstoffe sein. Ferner sind verschiedene thermoplastische Elastomere denkbar.

Es versteht sich, dass die genutzten elastomeren Werkstoffe in geeigneter Weise mit Additiven, Beschichtungen und/oder Oberflächenbehandlungen versehen sein können, um sie noch weiter für die angestrebte Verwendung zu ertüchtigen. Insbesondere ist es bevorzugt, wenn das Formteil durch ein urformendes Fertigungsverfahren erzeugbar ist. Dabei kann es sich insbesondere um ein zumindest endkonturnahes Fertigungsverfahren handeln. Auf diese Weise können auch relativ komplexe Formteile mit geringem Aufwand hergestellt werden. Insbesondere ist es bevorzugt, wenn das Formteil derart gestaltet ist, dass der in diesem ausgebildete zumindest eine Strömungspfad in einem unbelasteten Zustand im Wesentlichen konstante Strömungsquerschnitte bereitstellt.

Bei dem Aktor kann es sich insbesondere um einen Magnetaktor handeln. Demgemäß kann der Aktor als elektromagnetischer Aktor ausgebildet sein und einen beweglichen Anker umfassen, vorzugsweise einen Schwenkanker, der in einem aktivierten Zustand auf das zumindest eine Sperrglied einwirkt. In alternativer Weise kann der Aktor einen Zuganker umfassen. Der Aktor kann dazu ausgestaltet sein, beim Übergang zwischen einem inaktiven und einem aktiven (bestromten) Zustand einen definierten Hub bzw. eine definierte Kraft bereitzustellen.

Das zumindest eine Sperrglied kann etwa nach Art einer Schlauchklemme von außen auf den zu sperrenden Leitungsabschnitt einwirken. Auf diese Weise kann der Leitungsabschnitt elastisch verformt werden, so dass der im Leitungsabschnitt ausgebildete Strömungsabschnitt in der Sperrstellung geschlossen bzw. gesperrt wird, um einen Durchfluss des Mediums zu unterbinden.

Erfindungsgemäß weist das Formteil drei oder mehr Anschlüsse auf, zwischen denen sich zumindest zwei Strömungspfade erstrecken, wobei der Aktor mit zumindest zwei Sperrgliedern gekoppelt ist, um jeweils zumindest einen Strömungspfad der zumindest zwei Strömungspfade selektiv zu sperren oder freizugeben. Mit anderen Worten kann die Vorrichtung als 3-Wegeventil gestaltet sein. Die Anschlüsse können beispielsweise als COM, NC und NO bezeichnet werden. Bei dem mit COM bezeichneten Anschluss kann es sich um einen gemeinsamen Anschluss handeln, etwa um eine gemeinsame Zuleitung oder Ableitung. Der NC-Anschluss (normally closed) ist regelmäßig der Anschluss, der in einem inaktiven Zustand des Aktors gesperrt ist. Der NO-Anschluss (normally open) ist regelmäßig der Anschluss, der in einem Ruhezustand des Aktors geöffnet ist. Insgesamt kann die Vorrichtung somit zumindest zwei diskrete Schaltstellungen aufweisen, in denen entweder der Strömungspfad zwischen den Anschlüssen COM und NC oder der Strömungspfad zwischen den Anschlüssen COM und NO definiert gesperrt ist. Es versteht sich, dass auch zumindest ein dritter Zustand vorstellbar ist, bei dem beide Strömungspfade gesperrt sind.

Erfindungsgemäß weist die Vorrichtung ferner einen Koppelmechanismus auf, der durch den Aktor antreibbar ist, wobei der Koppelmechanismus das erste Sperrglied und das zweite Sperrglied mechanisch miteinander koppelt, wobei sich das erste Sperrglied in der Sperrstellung befindet, wenn sich das zweite Sperrglied in der Freigabestellung befindet, und wobei sich das erste Sperrglied in der Freigabestellung befindet, wenn sich das zweite Sperrglied in der Sperrstellung befindet. Auf diese Weise kann mittels lediglich einem Aktor eine Mehrzahl von diskreten Zuständen bewirkt werden. Ferner kann eine Zwangskopplung realisiert werden, so dass die Funktionssicherheit und Ausfallsicherheit der Vorrichtung erhöht werden kann.

Für die Sperrglieder kann ferner etwa gehäuseseitig bei der Vorrichtung ein Anschlag vorgesehen sein, um Überlastungen bzw. übermäßige Beanspruchungen insbesondere in der Sperrstellung zu vermeiden. Auf diese Weise kann etwa eine übermäßige Deformation des Formteils oder eine übermäßige Auslenkung der Sperrglieder vermieden werden.

Das erste Sperrglied und das zweite Sperrglied sind derart miteinander gekoppelt, dass das erste Sperrglied und das zweite Sperrglied zumindest in einer Zwischenstellung zwischen der Sperrstellung des ersten Sperrgliedes und der Sperrstellung des zweiten Sperrgliedes die diesen zugeordneten Strömungspfade gleichzeitig sperren. Auf diese Weise kann eine dritte definierte Stellung bzw. ein dritter Schaltzustand bewirkt werden, in dem beide Strömungspfade gesperrt sind. Dies hat den wesentlichen Vorteil, dass der Übergang zwischen den Schaltzuständen, bei denen jeweils ein Strömungspfad freigegeben ist, überschneidungsfrei erfolgen kann. Dies kann die Betriebssicherheit und etwa die Dosiergenauigkeit weiter erhöhen. Ferner kann ein Übertritt des Mediums aus einem Strömungspfad in einen anderen Strömungspfad wirksam unterbunden werden.

Mit anderen Worten ist es bevorzugt, wenn die Vorrichtung etwa nach Art einer Schleuse ein sich in der Sperrstellung befindliches Sperrglied erst dann in die Freigabestellung überführt, wenn das andere Sperrglied aus seiner Freigabestellung in die Sperrstellung geführt ist.

Diese Ausgestaltung kann auch Gegenstand einer eigenständigen Erfindung sein und demgemäß zur Anwendung kommen, ohne dass es eines Formteils gemäß den obigen Aspekten bedarf. Es empfiehlt sich jedoch, einen derartigen Koppelmechanismus mittelbar mit den jeweiligen Strömungspfaden bzw. Leitungsabschnitten zu koppeln, insbesondere unter Zwischenschaltung deformierbarer, hinreichend elastomerer Werkstoffe. Sofern nämlich der Koppelmechanismus mit Sperrgliedern gekoppelt ist, die mittelbar über deformierbare Werkstoffe die Strömungspfade sperren können, sinkt die Anforderung an Fertigungstoleranzen und/oder Montagetoleranzen, so dass die gewünschte Funktionalität auch erzielt wird, wenn die Sperrglieder lediglich hinreichend genau positioniert bzw. bewegt werden.

Gemäß einer weiteren Ausgestaltung weist die Vorrichtung ferner ein Vorspannelement auf, das mit dem Koppelmechanismus gekoppelt ist und das erste Sperrglied und das zweite Sperrglied zumindest in der Zwischenstellung zwischen der Sperrstellung des ersten Sperrgliedes und der Sperrstellung des zweiten Sperrgliedes in Richtung auf die diesem zugeordneten Leitungsabschnitte beaufschlagt bzw. drängt. Somit kann das Vorspannelement in der Zwischenstellung auf die Sperrglieder einwirken, um zumindest abschnittsweise beide Leitungsabschnitte zu sperren. Dies kann den gewünschten überschneidungsfreien Übergang herbeiführen. Das Vorspannelement kann etwa auch als Absperrfeder bezeichnet werden.

Gemäß einer weiteren Ausgestaltung ist zumindest ein Sperrglied des Koppelmechanismus über ein Stoffgelenk mit einem Gehäuseteil der Vorrichtung gekoppelt. Mit anderen Worten kann das Sperrglied als integraler Bestandteil des Gehäuseteils gefertigt sein. Dies kann den Fertigungsaufwand und insbesondere den Montageaufwand weiter reduzieren. Das Sperrglied kann über das Stoffgelenk schwenkbar am Gehäuseteil gelagert sein. Dies kann weiter zur Miniaturisierung der Vorrichtung beitragen.

In einer bevorzugten Ausgestaltung ist der Koppelmechanismus als Scherenmechanismus ausgestaltet, wobei das erste Sperrglied einen ersten Scherenhebel und das zweite Sperrglied einen zweiten Scherenhebel zugeordnet ist, die vorzugsweise gelenkig miteinander gekoppelt sind. Diese Maßnahme hat den Vorteil, dass mittels lediglich eines Aktors auf einen der beiden Scherenhebel eingewirkt werden kann, wobei die Kopplung zwischen den beiden Scherenhebeln eine entsprechende Bewegung des zweiten Scherenhebels induziert. Vorzugsweise ist zumindest einer der beiden Scherenhebel stoffschlüssig an einem Gehäuseteil der Vorrichtung aufgenommen.

Gemäß einer weiteren Ausgestaltung des als Scherenmechanismus ausgestalteten Koppelmechanismus sind das erste Sperrglied und das zweite Sperrglied relativ zueinander vorgespannt, um eine Relativverschwenkung zwischen dem ersten Sperrglied und dem zweiten Sperrglied zu bewirken, wobei vorzugsweise der erste Scherenhebel in Richtung auf einen ersten Anschlag und der zweite Scherenhebel in Richtung auf einen zweiten Anschlag am Aktor, insbesondere an einem Anker des Aktors, beaufschlagt ist. Mit anderen Worten kann ein Vorspannelement vorgesehen sein, das zwischen den beiden Scherenhebeln des Scherenmechanismus aufgenommen ist und gemeinsam mit den beiden Anschlägen eine definierte Relativlage zwischen den Scherenhebeln und somit den Sperrgliedern herbeiführt. Dies kann die Schaltgenauigkeit und -präzision weiter erhöhen. Insbesondere kann diese Maßnahme dazu beitragen, dass der Übergang zwischen den beiden Extremlagen, in denen sich jeweils ein Sperrglied in der Sperrstellung befindet, überschneidungsfrei erfolgt. Zu diesem Zweck kann das Vorspannelement insbesondere mittelbar oder unmittelbar auf eine Schwenkachse des Scherenmechanismus einwirken.

Gemäß einer weiteren Ausgestaltung ist der Koppelmechanismus als Kipphebelmechanismus ausgestaltet und mit einem Kipphebel versehen, der eine starre Verbindung zwischen dem ersten Sperrglied und dem zweiten Sperrglied aufweist und um eine Schwenkachse verschwenkbar ist, um das erste Sperrglied und das zweite Sperrglied wechselseitig in die Sperrstellung zu überführen. Gemäß dieser Ausgestaltung kann der Koppelmechanismus grundsätzlich nach Art einer (Doppel-)Wippe gestaltet sein.

Gemäß einer Weiterbildung dieser Ausgestaltung ist der Kipphebel über ein Langloch längsverschieblich und verschwenkbar mit der Schwenkachse gekoppelt, wobei der Kipphebel in Richtung auf das Formteil vorgespannt ist, wobei der Kipphebel vorzugsweise über eine Längsverschiebung relativ zur Schwenkachse bewegbar ist. In diesem Zusammenhang ist es weiter bevorzugt, wenn ein Vorspannelement den Kipphebel in Richtung auf das Formteil beaufschlagt. Somit kann der Kipphebel einerseits durch den Aktor verschwenkt werden, um wechselseitig das erste Sperrglied und das zweite Sperrglied in die Sperrstellung zu drängen. Während des Übergangs zwischen den beiden Extremlagen wird jedoch durch das Vorspannelement sichergestellt, dass ein überschneidungsfreier Übergang gewährleistet ist. Zu diesem Zweck ist der Kipphebel über die Paarung Langloch-Schwenkachse zumindest minimal "auslenkbar", um beide Sperrglieder gleichzeitig sperrend in einen Eingriff mit dem Formteil bringen zu können.

Gemäß einer weiteren Ausgestaltung weist die Vorrichtung ferner ein Rückstellelement auf, insbesondere eine Rückstellfeder, wobei das Rückstellelement den Koppelmechanismus in Richtung auf eine Ruhestellung beaufschlagt, wenn der Aktor inaktiv ist. In der Ruhestellung kann sich etwa zwischen dem COM-Anschluss und dem NO-Anschluss ein Durchfluss ergeben, wobei sich der sich zwischen dem COM-Anschluss und dem NC-Anschluss erstreckende Strömungspfad gesperrt ist. Demgemäß kann sich das erste Sperrglied in der Sperrstellung befinden.

Gemäß einer weiteren Ausgestaltung der Vorrichtung ist das Formteil als auswechselbares Einlegeteil gestaltet, wobei das Formteil vorzugsweise als Formschlauch auf Basis eines Silikonwerkstoffs oder eines Fluorkunststoffs gestaltet ist, und wobei Tüllen an das Formteil angeformt sind, die vorzugsweise den Anschlüssen benachbart sind. Die Tüllen können einerseits eine Anschlussgeometrie für externe Leitungen bereitstellen. Ferner können die Tüllen eine Ausrichtung des Formteils an der Vorrichtung gewährleisten. Somit kann das Formteil in einfacher Weise ausgewechselt werden, wobei sich eine Soll-Lage und Soll-Ausrichtung des Formteils gewissermaßen zwangsläufig durch die Anordnung und Ausgestaltung der Tüllen ergeben kann.

Gemäß einer weiteren Ausgestaltung weist die Vorrichtung ferner zumindest ein erstes Gehäuseteil und ein zweites Gehäuseteil auf, die vorzugsweise über eine Schnappverbindung miteinander rastend verbindbar sind. Der Aktor kann am ersten Gehäuseteil aufgenommen sein. Eines der Gehäuseteile kann eine definierte Aufnahmekontur zur formschlüssigen Aufnahme des Formkörpers auweisen. Vorzugsweise ist das erste Gehäuseteil oder das zweite Gehäuseteil, weiter bevorzugt beide Gehäuseteile, aus einem Kunststoffwerkstoff hergestellt. Hierbei kann es sich etwa um Polyoxymethylen (POM), Polyamid (PA), Polymethylmethacrylat (PMMA) oder ähnliche Kunststoffwerkstoffe handeln, die sich insbesondere zur Verarbeitung mittels Spritzguss eignen. Vorzugsweise ist das Formteil aus einem sterilisierbaren Werkstoff gestaltet. In einfacher Weise kann das Formteil entfernt werden, um es einem Reinigungsvorgang oder gar einer Sterilisierung zuzuführen.

Vorzugsweise ist das zweite Gehäuseteil einfach zugänglich, um das Auswechseln des Formkörpers weiter zu vereinfachen. Da die potentiell aggressiven oder hochempfindlichen Medien vollständig durch das Formteil von den Gehäuseteilen separiert sind, können die Gehäuseteile aus günstigen und einfach verarbeitbaren Werkstoffen gefertigt sein. Insbesondere ist es nicht erforderlich, in den Gehäuseteilen Strömungskanäle zur Strömungsführung bereitzuhalten, die aus Edelstahl gebildet sind. Am zweiten Gehäuseteil kann ferner zumindest ein Anschlag zur Begrenzung der Auslenkung zumindest eines der Sperrglieder, vorzugsweise beider Sperrglieder, ausgebildet sein.

Gemäß einer weiteren Ausgestaltung der Vorrichtung ist der Aktor als elektromagnetischer Aktor ausgebildet. Der Aktor kann ein C-förmiges oder U-förmiges Joch aufweisen, das mit einem Anker gekoppelt ist, vorzugsweise mit einem Schwenkanker oder Klappanker. Das Joch kann mit einer Mehrzahl von Spulen gekoppelt sein, vorzugsweise mit drei Spulen, von denen jede einem Schenkel bzw. Abschnitt des Jochs zugeordnet sein kann. Das C-förmige oder U-förmige Joch weist beispielhaft drei Schenkel auf, von denen jedes eine Spule aufnehmen kann. Auf diese Weise kann bei gegebenen Maximalabmessungen des Aktors die Magnetkraft maximiert werden.

Es versteht sich, dass auf Basis einer Vorrichtung, die gemäß zumindest einigen der oben genannten Aspekte gestaltet ist, eine Ventilanordnung erzeugbar ist, die mehrere Aktoren und gegebenenfalls auch mehrere Formteile aufweist. Es versteht sich, dass auch ein komplex gestaltetes Formteil vorgesehen sein kann, das mehr als drei Anschlüsse und entsprechende Strömungspfade aufweist. Ein solches Formteil kann mit einer Mehrzahl von Sperrgliedern gekoppelt sein, die in entsprechender Weise durch eine Mehrzahl von Aktoren ansteuerbar sind.

Die Verwendung betreffend wird die Aufgabe der Erfindung durch eine Verwendung eines Formteils aus einem elastischen Werkstoff als auswechselbares Einlegeteil in einer Vorrichtung zur Steuerung fluidischer Medien nach Anspruch 1 gelöst.

Vorteilhaft erfolgt die Verwendung bei einer Vorrichtung zur Steuerung fluidischer Medien, die gemäß zumindest einem der vorstehend beschriebenen Aspekte ausgestaltet ist.

Auch auf diese Weise wird die Aufgabe der Erfindung vollkommen gelöst.

Das Formteil eignet sich als auswechselbares Einlegeteil für ein Wegeventil, insbesondere ein Mehrwegeventil. In einfacher Weise kann das Formteil ausgetauscht werden, um etwa auf einen Verschleiß oder eine Beschädigung reagieren zu können, ferner kann die Vorrichtung in einfacher Weise durch Auswechseln des Formteils an verschiedene Medien angepasst werden, ohne dass es einer aufwendigen Reinigung oder gar Sterilisation bedarf.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Merkmale und Vorteile der Erfindung ergeben sich aus der nachfolgenden Beschreibung mehrerer bevorzugter Ausführungsbeispiele unter Bezugnahme auf die Zeichnung. Es zeigen:
- Fig. 1: einen Längsschnitt durch eine beispielhafte Ausgestaltung einer Vorrichtung zur Steuerung fluidischer Medien;
- Fig. 2: eine perspektivische Explosionsansicht der Vorrichtung gemäß Fig. 1;
- Fig. 3: einen schematisch stark vereinfachten seitlichen Schnitt durch einen Leitungsabschnitt, der mittels eines Sperrglieds absperrbar ist, wobei sich das Sperrglied in einer Freigabestellung befindet;
- Fig. 4: eine weitere Ansicht des Leitungsabschnitts gemäß Fig. 3, wobei sich das Sperrglied in einer Sperrstellung befindet;
- Fig. 5: einen Längsschnitt durch eine alternative beispielhafte Ausgestaltung einer Vorrichtung zur Steuerung fluidischer Medien; und
- Fig. 6: eine perspektivische Explosionsansicht der Vorrichtung gemäß Fig. 5.

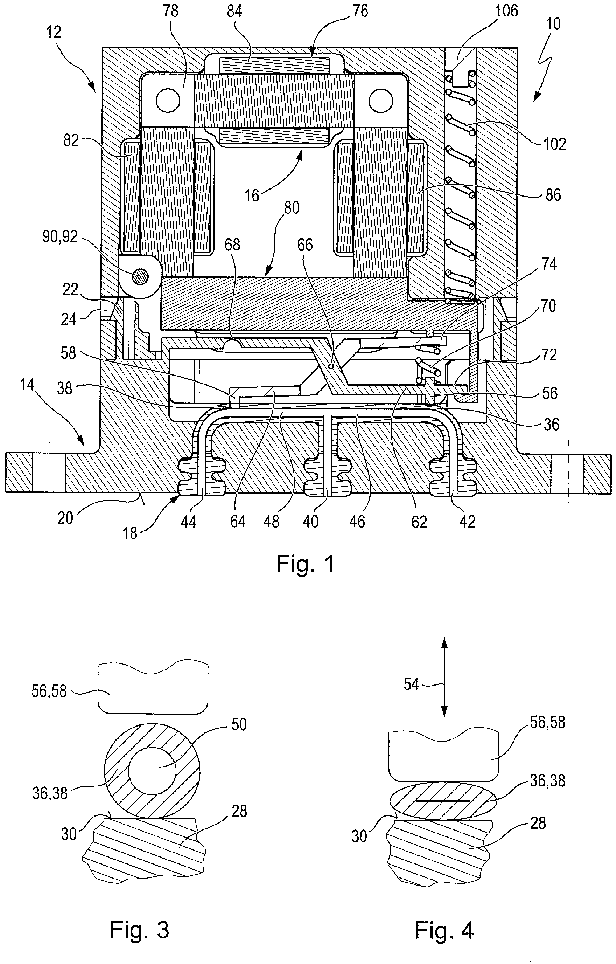

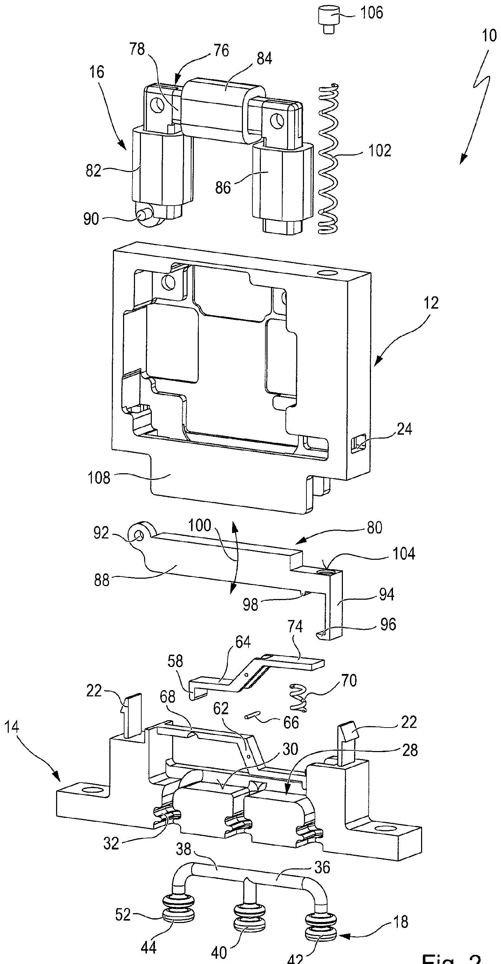

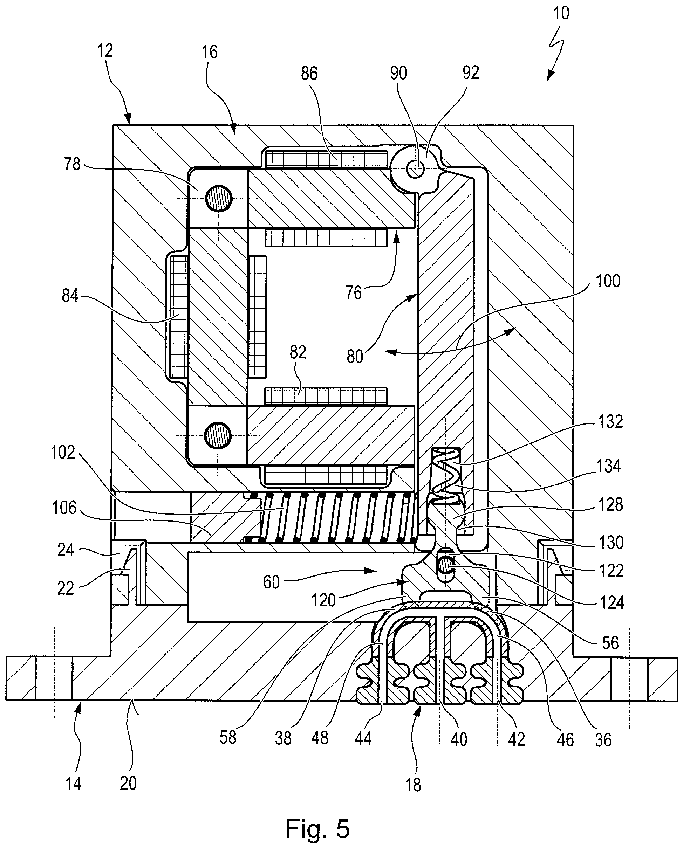

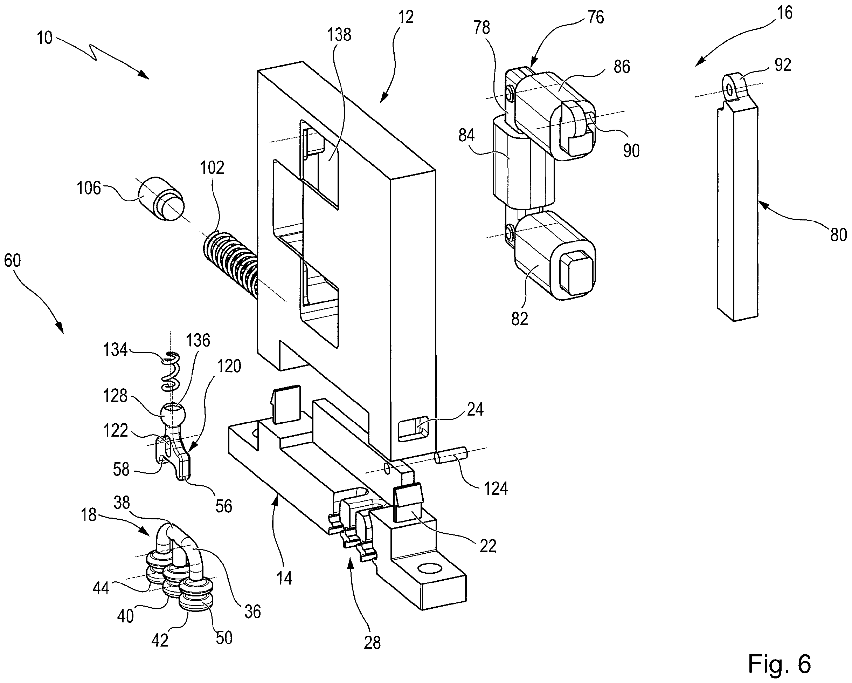

In Fig. 1 ist eine Vorrichtung 10 zur Steuerung fluidischer Medien anhand eines Längsschnittes veranschaulicht. Eine mit der Gestaltung der Vorrichtung gemäß Fig. 1 korrespondierende Explosionsansicht perspektivischer Art ist Fig. 2 entnehmbar. Eine alternative Ausgestaltung einer Vorrichtung 10 zur Steuerung fluidischer Medien kann dem Längsschnitt gemäß Fig. 5 sowie der korrespondierenden Explosionsdarstellung gemäß Fig. 6 entnommen werden. Die Vorrichtungen 10 können insbesondere als Ventilanordnungen ausgebildet sein, vorzugsweise als sogenannte Wegeventile. Beispielhaft sind die Vorrichtungen 10 der Ausgestaltungen gemäß den Fig. 1, 2 sowie 5, 6 als sogenannte Zahl 3/2-Wegeventile ausgestaltet. Mit anderen Worten weist jede Vorrichtung 10 drei Anschlüsse sowie zwei definierte Schaltstellungen auf. Andere Gestaltungen sind ohne Weiteres denkbar und können sowohl die Zahl der Anschlüsse bzw. der sich zwischen den Anschlüssen erstreckenden Fluidpfade sowie die Anzahl denkbarer Schaltstellungen betreffen.

Den Fig. 1 und 2 kann entnommen werden, dass die Vorrichtung 10 ein erstes Gehäuseteil 12 und ein zweites Gehäuseteil 14 aufweist. Das erste Gehäuseteil 12 ist insbesondere zur Aufnahme einer Steuereinheit 16 ausgebildet, die beispielsweise einen magnetischen Aktor aufweisen kann. Das zweite Gehäuseteil 14 ist insbesondere zur Aufnahme eines Formteils 18 ausgestaltet, das etwa als sogenanntes Schlaucheinlegeteil ausgeführt ist. In zumindest einigen Ausgestaltungen kann das erste Gehäuseteil 12 als Oberteil und das zweite Gehäuseteil 14 als Unterteil bezeichnet werden. Es versteht sich, dass die entsprechenden Zuordnungen lediglich exemplarischer Natur sind und nicht einschränkend zu verstehen sind. Am zweiten Gehäuseteil 14 ist eine Schnittstelle 20 zur Montage oder Verbindung der Steuervorrichtung 10 ausgebildet. Mit anderen Worten kann die Schnittstelle 20 etwa eine Anschlussplatte umfassen.

Vorzugsweise ist zumindest das erste Gehäuseteil 12 oder das zweite Gehäuseteil 14 als Gußteil aus einem mittels Spritzguss verarbeitbaren Werkstoff gefertigt, insbesondere aus einem Thermoplast. Auf diese Weise können die Gehäuseteile 12, 14 einfach und kostengünstig in großen Stückzahlen hergestellt werden. In einer bevorzugten Ausgestaltung sind die Gehäuseteile 12, 14 über eine Schnappverbindung miteinander gefügt. Zu diesem Zweck ist an einem der Gehäuseteile 12, 14 zumindest ein Schnapphaken 22 ausgebildet, der in eine Rastausnehmung 24 eingreifen kann, die am anderen Gehäuseteil 12, 14 vorgesehen ist. Auf diese Weise können die Gehäuseteile 12, 14 mittels einer Hinterschnappverbindung sicher gefügt werden. Es versteht sich, dass das Gehäuse der Steuervorrichtung 10 auch auf andere Weise gestaltet sein kann und etwa einstückig, zweistückig, dreistückig gebildet sein kann. Ferner kann sich auch eine von der Darstellung gemäß Fig. 1 abweichende Zuordnung der Steuereinheit 16 sowie des Formteils 18 zu den Gehäuseteilen 12, 14 ergeben. Gleichwohl ist die Gestaltung gemäß den Fig. 1 und 2 Gegenstand bevorzugter Ausführungsformen.

Fig. 2 zeigt, dass das zweite Gehäuseteil 14 mit einer Aufnahmekontur 28 für das Formteil 18 versehen ist. Die Aufnahmekontur 28 umfasst eine Einlegekontur, in die das Formteil 18 definiert einfügbar bzw. einlegbar ist. Die Aufnahmekontur 28 weist zumindest ein Flachstück 30, vorzugsweise zwei Flachstücke 30 und zumindest eine Klemmnut oder Aufnahmenut 32 auf. Insgesamt kann die Aufnahmekontur 28 zumindest abschnittsweise als "Negativ" der Kontur des Formteils 18 gefertigt sein. Aus Fig. 2 ist ferner ersichtlich, dass das Formteil 18 seitlich in die Aufnahmekontur 28 beim zweiten Gehäuseteil 14 eingeführt bzw. eingefügt werden kann. Insgesamt kann das Formteil 18 auf diese Weise einfach und schnell ausgewechselt werden. Dies kann im Reparaturfall bzw. Verschleißfall von Vorteil sein. Dies kann ferner von Vorteil sein, wenn eine Mehrzahl von Formteilen 18 vorgehalten wird, um verschiedene Fluide bzw. Medien regeln zu können. Auf diese Weise kann die Steuervorrichtung 10 zur Steuerung bzw. Regelung verschiedenartiger Medien genutzt werden, die insbesondere auch hochreine, empfindliche, aggressive, hochaggressive, reaktive, hochreaktive sowie allgemein reaktionsfreudige Medien umfassen können.

Fig. 2 zeigt ferner, dass das Formteil 18 im Wesentlichen etwa W-förmig bzw. m-förmig gestaltet ist, wobei beispielhaft ein erster Leitungsabschnitt 36 und ein zweiter Leitungsabschnitt 38 vorgesehen sind. Der erste Leitungsabschnitt 36 erstreckt sich zwischen einem ersten Anschluss 40, der auch als COM-Anschluss bezeichnet werden kann, und einem zweiten Anschluss 42, der auch als NC-Anschluss bezeichnet werden kann. Der zweite Leitungsabschnitt 38 erstreckt sich beispielhaft zwischen dem ersten Anschluss 40 und einem dritten Anschluss 44, der auch als NO-Anschluss bezeichnet werden kann. Die Anschlüsse 40, 42, 44 können jeweils einem Ende eines Schenkels des "W" bzw. "m" zugeordnet sein.

Jeweils zwischen zweien der Anschlüsse 40, 42, 44 kann ein Strömungspfad 46, 48 definiert sein. Beispielhaft erstreckt sich ein erster Strömungspfad 46 zwischen dem ersten Anschluss 40 und zweiten Anschluss 42, vgl. auch Fig. 1. Demgemäß erstreckt sich ein zweiter Strömungspfad 48 zwischen dem ersten Anschluss 40 und dem dritten Anschluss 44. Die Strömungspfade 46, 48 befinden sich vollständig in dem Formteil 18. Demgemäß kontaktiert keine andere Komponente der Steuervorrichtung 10 einen der Strömungspfade 46, 48 unmittelbar. Vorzugsweise ist das Formteil 18 derart gestaltet, dass die Strömungspfade 46, 48 einen zumindest im Wesentlichen konstanten Strömungsquerschnitt 50 aufweisen, vgl. hierzu etwa auch Fig. 3. Dies gilt insbesondere für einen neutralen, unbelasteten Zustand des Formteils 18.

Aus Fig. 2 ist ferner ersichtlich, dass zumindest einem oder jedem der Anschlüsse 40, 42, 44 eine Tülle 52 zugeordnet ist, die als integraler Bestandteil des Formteils 18 ausgebildet bzw. an dieses angeformt sein kann. Vorzugsweise ist die zumindest eine Tülle 52 derart an die zumindest eine Aufnahmenut 32 der Aufnahmekontur 28 angepasst, dass das Formteil 18 definiert und zumindest teilweise formschlüssig aufnehmbar ist. Zu diesem Zweck kann die zumindest eine Tülle 52 in die zumindest eine korrespondierende Aufnahmenut 32 eingeführt werden, wobei die Leitungsabschnitte 36, 38 an den Flachstücken 30 zur Anlage gelangen können.

Eine wesentliche Aufgabe der Steuervorrichtung 10 ist es, die Strömungspfade 46, 48, die durch das Formteil 18 definiert sind, selektiv freizugeben bzw. zu sperren. Zu diesem Zweck weist die Steuervorrichtung 10 beispielhaft ein erstes Sperrglied 56 und ein zweites Sperrglied 58 auf, die allgemein auch als Klemmen bezeichnet werden können. Beispielhaft ist das erste Sperrglied 56 dem ersten Leitungsabschnitt 36 zugeordnet. Demgemäß ist das zweite Sperrglied 58 dem zweiten Leitungsabschnitt 38 zugeordnet. Die Strömungspfade 46, 58 sind über die Sperrglieder 56, 58 mittelbar absperrbar. Dies bedeutet, dass die Sperrglieder 56, 58 nicht unmittelbar in Kontakt mit den strömenden Medien kommen müssen, um die Strömungspfade 46, 48 wirksam abzusperren.

Beispielhaft wird in diesem Zusammenhang auf die schematischen Darstellungen der Fig. 3 und 4 verwiesen. Fig. 3 zeigt eine sogenannte Freigabestellung eines Sperrglieds 56, 58. Fig. 4 zeigt eine sogenannte Sperrstellung eines Sperrglieds 56, 58. In der Freigabestellung gemäß Fig. 3 wirkt das Sperrglied 56, 58 nicht oder nur unwesentlich auf den Leitungsabschnitt 36, 38 ein. Demgemäß steht der Strömungsquerschnitt 50 vollständig oder nahezu vollständig zur Verfügung, das gewünschte Medium kann den entsprechenden Leitungsabschnitt 36, 38 durchströmen. In der anhand der Fig. 4 veranschaulichten Sperrstellung greift das Sperrglied 56, 58 an den Leitungsabschnitt 36, 38 an, um den Strömungsquerschnitt 50 zu schließen bzw. zu blockieren. Zu diesem Zweck kann das Sperrglied 56, 58 mit einer definierten Kraft auf den Leitungsabschnitt 36, 38 einwirken, um diesen so weit zu verformen, dass der Strömungsquerschnitt 50 (vgl. Fig. 3) möglichst dichtend geschlossen wird. Die Aufnahmekontur 28 bzw. deren Flachstück 30 fungieren dann als Gegenlager. Demgemäß kann das Sperrglied 56, 58 den Leitungsabschnitt 36, 38 in Richtung auf das Flachstück 30 beaufschlagen. Eine erforderliche Bewegung des Sperrglieds 56, 58 zwischen der Sperrstellung gemäß Fig. 4 und der Freigabestellung gemäß Fig. 3 ist in Fig. 4 durch einen mit 54 bezeichneten Doppelpfeil veranschaulicht. Zur Erzeugung dieser Bewegung bzw. der erforderlichen Kraft zum Sperren des gewünschten Strömungspfads 46, 48 ist die Steuereinheit 16 der Steuervorrichtung 10 mit den Sperrgliedern 56, 58 gekoppelt bzw. koppelbar.

Mit erneuter Bezugnahme auf die Fig. 1 und 2 wird eine beispielhafte Ausgestaltung eines Koppelmechanismus 60 veranschaulicht, der einerseits die Sperrglieder 56, 58 miteinander verbinden und andererseits eine Kopplung zwischen der Steuereinheit 16 und den Sperrgliedern 56, 58 bewirken kann. Gemäß der anhand der Fig. 1 und 2 veranschaulichten Ausgestaltung der Steuervorrichtung 10 kann es sich bei dem Koppelmechanismus 60 um einen sogenannten Scherenmechanismus handeln, der einen ersten Scherenhebel 62 und einen zweiten Scherenhebel 64 umfasst. Die Scherenhebel 62, 64 sind über eine Schwenkachse 66 miteinander gekoppelt. Zumindest einer der Scherenhebel 62, 64 kann stoffschlüssig mit zumindest einem der Gehäuseteile 12, 14 verbunden sein. Beispielhaft ist der erste Scherenhebel 62 über ein Stoffgelenk 68 mit dem zweiten Gehäuseteil 14 verbunden. Mit anderen Worten ist der erste Scherenhebel 62 als integraler Bestandteil des zweiten Gehäuseteils 14 gefertigt. Das Stoffgelenk 68 ist beispielhaft als bewusste Materialschwächung ausgebildet und erlaubt eine Relativbewegung des ersten Schwenkhebels 62 gegenüber dem zweiten Gehäuseteil 14.

Insbesondere der Darstellung gemäß Fig. 1 ist entnehmbar, dass der erste Scherenhebel 62 und der zweite Scherenhebel 64 über die Schwenkachse 66 gekreuzt miteinander koppelbar sind. Beispielhaft ist am ersten Scherenhebel 62 das erste Sperrglied 56 ausgebildet, das auf den ersten Leitungsabschnitt 36 einwirkt. Demgemäß ist am zweiten Scherenhebel 64 das zweite Sperrglied 58 ausgebildet, das auf den zweiten Leitungsabschnitt 38 einwirkt. Die Sperrglieder 56, 58 sind als wandartige Vorsprünge an den Scherenhebeln 62, 64 ausgebildet, die sich in Richtung auf die zugeordneten Leitungsabschnitte 36, 38 erstrecken. Die Sperrglieder 56, 58 können an ihren den Leitungsabschnitten 36, 38 zugewandten Enden zumindest angefast oder verrundet sein, um "sanft" auf die verformbaren Leitungsabschnitte 36, 38 einzuwirken.

Die Scherenhebel 62, 64 sind ferner über ein Vorspannelement 70 miteinander gekoppelt, das etwa als Druckfeder ausgebildet sein kann. Das Vorspannelement 70 wirkt bei der gemäß Fig. 1 gezeigten Ausgestaltung derart auf die Schwenkhebel 62, 64 ein, dass das erste Sperrglied 56 und das zweite Sperrglied 58 über entsprechende Schwenkbewegungen der Scherenhebel 62, 64 aufeinander zu bewegt werden würden, wenn nicht die Schwenkhebel 62, 64 an Kontaktabschnitten 72, 74 abgestützt und in einer hinreichend definierten Relativlage zueinander aufgenommen wären. Das Vorspannelement 70 ist dazu ausgebildet, derart auf die Scherenhebel 62, 64 einzuwirken, dass diese die Tendenz haben, sich gegenläufig zueinander um die Schwenkachse 66 zu verdrehen. Die spezielle Gestaltung des Koppelmechanismus 60 gemäß den Fig. 1 und 2 unterbindet jedoch eine übermäßige Relativverdrehung.

Die Steuereinheit 16 ist dazu ausgebildet, auf den Koppelmechanismus 60 einzuwirken, um selektiv zumindest einen der Leitungsabschnitte 36, 38 und damit zumindest einen der Strömungspfade 46, 48 zu sperren. Vorzugsweise weist die Steuereinheit 16 einen Aktor 76 auf, der insbesondere als elektromagnetischer Aktor ausgebildet sein kann. Der Aktor 76 weist ein Joch 78 auf, das mit einem Anker 80 zusammenwirkt. Am Joch 78 ist zumindest eine Spule 82, 84, 86 aufgenommen, über die der Aktor 76 aktivierbar bzw. bestrombar ist, um eine definierte Bewegung des Ankers 80 relativ zum Joch 78 herbeizuführen. Beispielhaft ist das Joch 78 insgesamt C-förmig bzw. U-förmig gestaltet, wobei insgesamt drei Spulen 82, 84, 86 vorgesehen sind, von denen etwa eine erste Spule 82 einen ersten Schenkel, eine zweite Spule 84 einer Basis sowie eine dritte Spule 86 einem zweiten Schenkel des Jochs 78 zugeordnet ist. Andere Gestaltungen sind ohne Weiteres denkbar. Es kann von Vorteil sein, eine Mehrzahl von Spulen 82, 84, 86 vorzusehen. Insgesamt kann somit auch bei einer begrenzten Bautiefe der Steuervorrichtung 10 (senkrecht zur Ansichtsebene in Fig. 1) eine beträchtliche Magnetkraft bewirkt werden. Auf diese Weise kann sich eine Gestaltung der Steuervorrichtung 10 ergeben, bei der die Gehäuseteile 12, 14 insgesamt eine etwa quaderförmige Gestalt aufweisen, bei der der Quader nur eine begrenzte Tiefenerstreckung aufweist.

Der Anker 80 weist einen Grundkörper 88 auf und ist insbesondere als sogenannter Schwenkanker gestaltet. Der Anker 80 weist ein Schwenklager 92 auf, das mit einer Schwenkachse 90 koppelbar ist, die dem Joch 78 des Aktors 76 zugeordnet ist, vgl. insbesondere auch Fig. 2. Eine denkbare Schwenkbewegung des Ankers 80 um sein Schwenklager 92 bzw. um die Schwenkachse 90 ist in Fig. 2 durch einen mit 100 bezeichneten Doppelpfeil veranschaulicht.

Der Aktor 80 ist mit dem Koppelmechanismus 60 gekoppelt, um in einem inaktiven (stromlosen) Zustand des Aktors 76 den ersten Strömungspfad 46 zwischen den Anschlüssen 40, 42 sowie in einem aktiven (bestromten) Zustand des Aktors 76 den zweiten Strömungspfad 48 zwischen den Anschlüssen 40, 44 zu sperren. Über den Koppelmechanismus 60 können somit mittels lediglich eines Aktors 76 zwei diskrete Zustände der Steuervorrichtung herbeigeführt werden. Es ist von Vorteil, wenn der Koppelmechanismus 60 ferner dazu ausgebildet ist, während des Übergangs zwischen diesen beiden Zuständen zumindest abschnittsweise einen Übergangszustand herbeizuführen, in dem beide Strömungspfade 46, 48 gleichzeitig gesperrt sind. Dies erlaubt einen überschneidungsfreien Übergang zwischen den beiden definierten Endzuständen.

Der Koppelmechanismus 60 ist in besonderer Weite dazu eingerichtet, sicherzustellen, dass auch während des Übergangs zwischen der Sperrstellung des ersten Sperrglieds 56 und der Sperrstellung des zweiten Sperrglieds 58 sich beide Sperrglieder 56, 58 zumindest kurzzeitig gleichzeitig in oder nahe ihrer Sperrstellung befinden, um gleichzeitig beide Strömungspfade 46, 48 zu sperren.

Allgemein ist der Grundkörper 88 des Ankers 80 etwa nach Art eines Riegels bzw. einer Stange gestaltet, wobei der Riegel dazu ausgebildet ist, die beiden Schenkel des U oder C des Jochs 78 an ihren offenen Enden miteinander zu verbinden, wenn sich der Aktor 76 im bestromten Zustand befindet. Darüber hinaus weist der Anker 80 gemäß der anhand der Fig. 1 und 2 veranschaulichten Ausgestaltung einen Haltesteg 94 auf, der an dem Ende des Grundkörpers 88 ausgebildet ist, das dem Schwenklager 92 abgewandt ist. Am Haltesteg 94 ist ein erster Anschlag 96 ausgebildet, der im gefügten Zustand den ersten Kontaktabschnitt 72 des ersten Scherenhebels 62 kontaktiert. Der erste Anschlag 96 ist dem riegelartigen Grundkörper 88 des Ankers 80 zugewandt. Am Grundkörper 88 ist ein zweiter Anschlag 98 ausgebildet, der dem ersten Anschlag 96 gegenüberliegend angeordnet ist. Der zweite Anschlag 98 kontaktiert im gefügten Zustand den am zweiten Scherenhebel 64 ausgebildeten Kontaktabschnitt 74.

Mit anderen Worten wirken der erste Kontaktabschnitt 72 und der zweite Kontaktabschnitt 74 als Klemme oder Klammer, wobei das Vorspannelement 70 die Kontaktabschnitte 72, 74 und somit den ersten Scherenhebel 62 und den zweiten Scherenhebel 64 auseinander drängt und zur Anlage an die Anschläge 96, 98 bringt. Diese Gestaltung bewirkt eine vorteilhafte Kopplung der Scherenhebel 62, 64. Wie vorstehend bereits ausgeführt, sind die Scherenhebel 62, 64 kreuzartig miteinander gekoppelt, etwa X-förmig (ähnlich einem Andreaskreuz). Die durch ein Aktivieren oder Deaktivieren des Aktors 76 bewirkbare Schwenkbewegung oder Auslenkung des Ankers 80 (Bezugszeichen 100 in Fig. 2) bewirkt im inaktiven Zustand ein Abheben des Ankers 80 von einer entsprechenden Kontaktfläche des Jochs 78. Diese Bewegung (gemäß der Gestaltung in Fig. 1 etwa eine Verdrehung im Uhrzeigersinn) wird über den Anschlag 98 auf den zweiten Kontaktabschnitt 74 des zweiten Scherenhebels 64 übertragen und sorgt dafür, dass der zweite Scherenhebel 64 derart verschwenkt wird, dass das zweite Sperrglied 58 vom zweiten Leitungsabschnitt 78 weg bewegt wird. Bei der Gestaltung gemäß Fig. 1 erfolgt die Schwenkbewegung des zweiten Scherenhebels 64 im Uhrzeigersinn.

Über das Vorspannelement 70 wird die Auslenkung bzw. Verlagerung des Ankers 80 über den ersten Kontaktabschnitt 72 auf den ersten Scherenhebel 62 übertragen. Dies führt dazu, dass das erste Sperrglied 56 in Richtung auf den ersten Leitungsabschnitt 36 bewegt wird, um diesen schlussendlich sperren zu können. Demgemäß würde sich bei der Gestaltung gemäß Fig. 1 auch der erste Schwenkhebel 62 im Uhrzeigersinn um die Schwenkachse 66 verdrehen, wenn der Anker 80 vom Joch 78 abhebt. Diese Bewegung, die etwa dann erfolgt, wenn der Aktor 78 deaktiviert wird bzw. stromlos geschaltet wird, wird durch ein Rückstellelement 102 unterstützt, das insbesondere als Rückstellfeder gestaltet ist. Das Rückstellelement 102 beispielsweise am ersten Gehäuseteil 12 aufgenommen, insbesondere in einer Ausnehmung oder Bohrung, die durch einen Stopfen 106 verschließbar ist. Das Rückstellelement 102 stützt sich am Stopfen 106 ab. Das Rückstellelement 102 ist beispielhaft als Druckfeder ausgeführt.

Das Rückstellelement 102 beaufschlagt eine Kraftangriffsfläche 104, die am Anker 80 ausgebildet ist. Demgemäß kann das Rückstellelement eine definierte Neutrallage des Ankers 80 bzw. des Koppelmechanismus 60 bewirken, wenn der Aktor 76 inaktiv (stromlos) ist. Sofern der Aktor 76 aktiviert (bestromt) wird, muss die Rückstellkraft, mit der das Rückstellelement 102 auf den Anker 80 einwirkt, überwunden werden, um den Anker 80 zur Anlage am Joch 78 zu bringen . Diese Bewegung des Ankers würde bei der Gestaltung gemäß Fig. 1 im Gegenuhrzeigersinn erfolgen. Eine solche Bewegung des Ankers 80 würde auch eine entsprechende Bewegung des ersten Scherenhebels 62 und des zweiten Scherenhebels 64 relativ zu deren Schwenkachse bewirken. Demgemäß würde das erste Sperrglied 56 aus seiner Sperrstellung in eine Freigabestellung überführt werden. Umgekehrt würde das zweite Sperrglied 58 aus seiner Freigabestellung in die Sperrstellung überführt werden, um den zweiten Leitungsabschnitt 38 zu sperren. Die besondere Gestaltung des Koppelmechanismus 60 gemäß Fig. 1 und 2 bewirkt jedoch ferner, dass sich während dieses Übergangs beide Leitungsabschnitte 36, 38 und somit beide Strömungspfade 46, 48 zumindest kurzzeitig gleichzeitig sperren lassen. Da nämlich der erste Scherenhebel 62 über ein Stoffgelenk 68 (und gerade nicht über die Schwenkachse 66) verschwenkbar am zweiten Gehäuseteil 14 aufgenommen ist, verschwenkt sich der erste Scherenhebel 62 bei der Bewegung des Ankers 80 nicht nur um die Schwenkachse 66, sondern eben auch um das Stoffgelenk 68 bzw. um eine durch das Stoffgelenk 68 bereitgestellte "virtuelle" Schwenkachse. Dies bewirkt, dass die Schwenkachse 66 bei der Bewegung des Ankers 80 gemeinsam und gleichsinnig mit diesem (aber nicht unbedingt betragsmäßig gleich) verschwenkt bzw. verlagert wird.

Fig. 1 veranschaulicht insbesondere einen aktiven (bestromten) Zustand des Aktors, bei dem der Anker 80 das Joch 78 kontaktiert. Demgemäß befindet sich in diesem Zustand die Schwenkachse 66 in einer vom Formteil 18 entfernten Stellung. Beim Übergang in den inaktiven (unbestromten) Zustand des Aktors, bei dem der Anker 80 vom Joch 78 abgehoben bzw. ausgelenkt ist, würde sich auch die Schwenkachse 66 relativ zum Stoffgelenk 68 verschwenken und dabei auf das Formteil 18 zu bewegt werden. Diese vorteilhafte Verschaltung bzw. die hieraus resultierende Verlagerbarkeit der Schwenkachse führt dazu, dass beim Übergang zwischen den beiden Extremlagen des Ankers 80 kurzzeitig beide Leitungsabschnitte 36, 38 durch die diesen zugeordneten Sperrglieder 56, 58 gesperrt werden. In vorteilhafter Weise kann dieser Zustand durch eine geeignete Auslegung und Gestaltung der Komponenten des Koppelmechanismus 60 bewirkt werden, ohne dass es etwa eines separaten Aktors 76 für diese Funktionalität bedarf.

Beispielhaft weist das erste Gehäuseteil 12 ferner zumindest eine seitliche Schürze oder Lasche 108 auf, die an dem dem zweiten Gehäuse 14 zugewandten Ende des ersten Gehäuseteils 12 angeordnet ist, vergleiche Fig. 2. Die Lasche 108 trägt zur Lagesicherung bzw. Lagefixierung des Formteils 18 am zweiten Gehäuseteil 14 bei. Ferner kann die Lasche 108 den Koppelmechanismus 60 zumindest hinreichend abdecken und vor Umgebungseinflüssen schützen.

Anhand der Fig. 5 und 6 wird eine alternative Ausgestaltung einer Steuervorrichtung 10 veranschaulicht, die im Hinblick auf ihre Funktionalität der anhand der Fig. 1 und 2 veranschaulichten Steuervorrichtung 10 weitgehend entspricht. Es wird daher nachfolgend vorrangig auf Unterscheidungsmerkmale eingegangen. Sofern keine expliziten Ausführungen zu einzelnen Komponenten der anhand der Fig. 5 und 6 veranschaulichten Ausgestaltung der Steuervorrichtung 10 erfolgen, können die Ausführungen zur Ausgestaltung gemäß den Fig. 1 und 2 ohne Weiteres übertragen werden.

In vorbeschriebener Weise ist die Steuervorrichtung 10 mit Gehäuseteilen 12, 14 versehen, wobei ein erstes Gehäuseteil 12 eine Steuereinheit 16 und ein zweites Gehäuseteil 14 ein Formteil 18 aufnehmen bzw. beherbergen können.

Auch die Steuervorrichtung 10 gemäß den Fig. 5 und 6 kann als Wegeventil gestaltet sein, insbesondere als 3/2-Wegeventil. Das Formteil 18 ist an einer Aufnahmekontur 28 des zweiten Gehäuseteils 14 aufgenommen und abgestützt. Das Formteil 18 weist beispielhaft zwei Leitungsabschnitte 36, 38 sowie drei Anschlüsse 40, 42, 44 auf, zwischen denen sich die Leitungsabschnitte 36, 38 erstrecken. Zwischen den Anschlüssen 40, 42 erstreckt sich ein erster Strömungspfad 46. Zwischen den Anschlüssen 40, 44 erstreckt sich ein zweiter Strömungspfad 48. Die Gehäuseteile 12, 14 können über eine Schnappverbindung, die zumindest einen Schnapphaken 22 und eine Rastausnehmung 24 aufweist, miteinander gefügt sein.

Die Steuereinheit 16 weist in grundsätzlich beschriebener Weise einen elektromagnetischen Aktor 76 auf, der ein C-förmiges oder U-förmiges Joch 78 umfasst, das mit Spulen 82, 84, 86 gekoppelt ist. Der Aktor 76 weist ferner einen Anker 80 auf, der relativ zum Joch 78 auslenkbar ist. Es kann sich beim Anker 80 insbesondere um einen Schwenkanker handeln, der um eine Schwenkachse 90 verschwenkbar ist, vergleiche auch den Doppelpfeil 100 in Fig. 5.

Bei der Gestaltung gemäß Fig. 5 erfährt der Anker 80 beim Übergang zwischen einem aktiven (bestromten) Zustand des Aktors 76 in einen inaktiven (unbestromten) Zustand eine Verschwenkung bzw. Auslenkung im Gegenuhrzeigersinn. Umgekehrt erfolgt beim Aktivieren (Bestromen) des Aktors eine entgegengerichtete Bewegung des Ankers 80 im Uhrzeigersinn, um das Joch 78 zu kontaktieren.

Auch bei der Gestaltung gemäß den Fig. 5 und 6 ist der Anker 80 dazu ausgebildet, auf einen Koppelmechanismus 60 einzuwirken, der mit Sperrgliedern 56, 58 versehen ist, die wahlweise den ersten Leitungsabschnitt 36 oder den zweiten Leitungsabschnitt 38 definiert sperren oder freigeben können. Wie vorstehend bereits ausgeführt, ist es auch beim Koppelmechanismus 60 gemäß den Fig. 5 und 6 von Vorteil, wenn während des Übergangs zwischen den beiden Schaltstellungen zumindest kurzzeitig beide Leitungsabschnitte 36, 38 bzw. die diesen zugeordneten Strömungspfade 46, 48 gleichzeitig gesperrt sind.

Der Koppelmechanismus 60 gemäß den Fig. 1 und 2 ist als Scherenmechanismus ausgestaltet. Im Gegensatz dazu ist der anhand der Fig. 5 und 6 veranschaulichte Koppelmechanismus 60 als Kipphebelmechanismus ausgeführt. Der Koppelmechanismus 60 weist einen Kipphebel 120 auf, der um eine Schwenkachse 124 verschwenkbar aufgenommen ist, die an zumindest einem der Gehäuseteile 12, 14, beispielsweise am zweiten Gehäuseteil 14, aufgenommen ist. Der Kipphebel 120 kann auch als Wippe bezeichnet werden. Der Kipphebel 120 wird gemäß der in Fig. 5 gezeigten Ausgestaltung im Uhrzeigersinn verschwenkt, um das erste Sperrglied 56 von seiner Freigabestellung in die Sperrstellung und gleichzeitig das zweite Sperrglied 58 von seiner Sperrstellung in Richtung auf seine Freigabestellung zu verlagern. Beide Sperrglieder 56, 58 sind am Kipphebel 120 ausgebildet, etwa als Vorsprünge, die sich in Richtung auf das Formteil 18 erstrecken.

Zur Aufnahme an der Schwenkachse 124 ist der Kipphebel 120 mit einer Ausnehmung versehen, die vorzugsweise als langlochartige Ausnehmung oder Langloch 122 gestaltet ist. Demgemäß ist der Kipphebel 120 nicht nur um die Schwenkachse 124 verschwenkbar, sondern auch entlang der Längserstreckung des Langlochs 122 relativ zur Schwenkachse 124 verschiebbar. Diese Gestaltung trägt dazu bei, dass die Steuervorrichtung 10 beim Übergang zwischen den beiden diskreten Schaltzuständen (entweder der erste Strömungspfad 46 und der zweite Strömungspfad 48 ist freigegeben) zumindest kurzzeitig beide Strömungspfade 46, 48 gleichzeitig sperrt.

Der Kipphebel 120 weist an seinem dem Formteil 18 abgewandten Ende einen Führungsabschnitt 128 auf, der etwa kopfartig, kugelartig oder kugelabschnittsartig gestaltet sein kann. Der Führungsabschnitt 128 ist in einer Führung 130 aufgenommen, die etwa als Zylinderführung oder zylindrische Ausnehmung in den Grundkörper 88 des Ankers 80 eingebracht ist. Ein Verschwenken des Ankers 80 im Gegenuhrzeigersinn bewirkt demgemäß ein Verschwenken des Kipphebels 120 im Uhrzeigersinn um die Schwenkachse 124. Diese Bewegung tritt beispielsweise dann auf, wenn der Aktor 76 deaktiviert, also etwa stromlos geschaltet wird. In bekannter Weise kann diese Bewegung durch ein Rückstellelement 102 unterstützt werden, das als Rückstellfeder gestaltet ist und den Anker 80 mit einer Rückstellkraft beaufschlagt.

Ferner weist der als Kipphebelmechanismus ausgebildete Koppelmechanismus 60 ein Vorspannelement 134 auf, das dem Anker 80 und dem Kipphebel 120 zwischengeschaltet ist. Das Vorspannelement 134 ist grundsätzlich dazu ausgestaltet, den Kipphebel 120 in Richtung auf einen Eingriff mit dem Formteil 18 zu beaufschlagen. Demgemäß bewirkt das Vorspannelement 134 eine Längsverschiebung des Kipphebels 120 relativ zur Schwenkachse 124 bewirken. Das Vorspannelement 134 ist in einer Federaufnahme 132 am Anker 80 aufgenommen, die beispielhaft als Verlängerung des Führungsabschnitts 128 in den Anker 80 eingebracht ist. Ferner ist am Führungsabschnitt 128 des Kipphebels 120 eine definierte Aufnahme 136 für das beispielhaft als Feder ausgebildete Vorspannelement 134 vorgesehen.

Demgemäß wird die reine Schwenkbewegung des Kipphebels 120 mit einer Längsverschiebung überlagert, um beide Sperrglieder 56, 58 in einer Zwischenstellung zwischen den definierten Schaltstellungen mit hinreichender Vorspannung in Kontakt mit den diesen zugeordneten Leitungsabschnitten 36, 38 zu bringen, um beide Strömungspfaden 46, 48 gleichzeitig zu sperren.

Fig. 6 ist ferner entnehmbar, dass beispielhaft das erste Gehäuseteil 12 mit definierten Aussparungen 138 versehen sein kann, von denen jede einer der Spulen 82, 84, 84 des Aktors 76 zugeordnet sein kann. Diese Maßnahme kann zum einen die Tiefenerstreckung oder Dickenerstreckung der Steuervorrichtung 10 weiter reduzieren. Ferner können die Aussparungen 138 dazu beitragen, Wärme effizient von den Spulen 82, 84, 86 abzuführen.

Es versteht sich, dass die im Rahmen dieser Offenbarung verwendeten Begriffe "im Uhrzeigersinn", "im Gegenuhrzeigersinn" und dergleichen jeweils auf eine Figur oder auf Figuren beziehen können, die eine beispielhafte Ausgestaltung zeigen. Demgemäß dürfen diese Begriffe nicht in einschränkender Weise verstanden werden. Es versteht sich, dass der Fachmann ohne Weiteres auf Basis der gezeigten Ausgestaltungen auch zu Ausgestaltungen und Ansichten gelangen kann, die etwa ein gegensinniges Layout aufweisen und somit zu gegensinnigen Bewegungen führen würden.

## Patentansprüche

1. Vorrichtung zur Steuerung fluidischer Medien in Form eines 3/2-Wegeventils mit drei Anschlüssen (40, 42, 44), mit einem Formteil (18) aus einem elastomeren Werkstoff, zwei Leitungsabschnitten (36, 38), die die drei Anschlüsse (40, 42, 44) miteinander verbinden, zwischen denen sich zwei Strömungspfade (46, 48) erstrecken, und mit einer Steuereinheit (16), die einen elektromagnetischen Aktor (76) umfasst, der mit zwei Sperrgliedern (56, 58) gekoppelt ist, wobei die zwei Sperrglieder (56, 58) durch den Aktor (76) zwischen einer Sperrstellung und einer Freigabestellung verlagerbar sind, wobei der Aktor (76) mit den zwei Sperrgliedern (56, 58) gekoppelt ist, um jeweils einen Strömungspfad (46; 48) der zwei Strömungspfade (46, 48) selektiv zu sperren oder freizugeben, wobei ein Koppelmechanismus (60) vorgesehen ist, der durch den Aktor (76) antreibbar ist, wobei der Koppelmechanismus (60) das erste Sperrglied (56) und das zweite Sperrglied (58) mechanisch miteinander koppelt, wobei sich das erste Sperrglied (56) in der Sperrstellung befindet, wenn sich das zweite Sperrglied (58) in der Freigabestellung befindet, und wobei sich das erste Sperrglied (56) in der Freigabestellung befindet, wenn sich das zweite Sperrglied (58) in der Sperrstellung befindet, **dadurch gekennzeichnet, dass** das erste Sperrglied (56, 58) in der Sperrstellung einen ersten Leitungsabschnitt (36, 38) verformt, um den ersten Strömungspfad (46, 48) zu sperren, dass das zweite Sperrglied (56, 58) in der Sperrstellung einen zweiten Leitungsabschnitt (36, 38) verformt, um den zweiten Strömungspfad (46, 48) zu sperren, dass die beiden Leitungsabschnitte (36, 38) in dem Formteil (18) ausgebildet sind, dass das Formteil (18) drei Anschlüsse (40, 42, 44) aufweist, zwischen denen sich die zwei Strömungspfade (46, 48) erstrecken, dass das Formteil (18) als auswechselbares Einlegeteil gestaltet ist, an das Tüllen (50) angeformt sind, die den Anschlüssen (40, 42, 44) benachbart sind und die Ausrichtung des Formteils (18) an der Vorrichtung gewährleisten, und dass das erste Sperrglied (56) und das zweite Sperrglied (58) derart miteinander gekoppelt sind, dass das erste Sperrglied (56) und das zweite Sperrglied (58) zumindest in einer Zwischenstellung zwischen der Sperrstellung des ersten Sperrgliedes (56) und der Sperrstellung des zweiten Sperrgliedes (58) die diesen zugeordneten Strömungspfade (46, 48) gleichzeitig sperren.

2. Vorrichtung nach Anspruch 1, **gekennzeichnet durch** ein Vorspannelement (70, 134), das mit dem Koppelmechanismus (60) gekoppelt ist und das erste Sperrglied (56) und das zweite Sperrglied (58) zumindest in der Zwischenstellung zwischen der Sperrstellung des ersten Sperrgliedes (56) und der Sperrstellung des zweiten Sperrgliedes (58) in Richtung auf die diesen zugeordneten Leitungsabschnitte (36, 38) beaufschlagt.

3. Vorrichtung nach einem der vorhergehenden Ansprüche, **dadurch gekennzeichnet, dass** zumindest ein Sperrglied (56, 58) des Koppelmechanismus (60) über ein Stoffgelenk (68) mit einem Gehäuseteil (14) der Vorrichtung gekoppelt ist.

4. Vorrichtung nach einem der vorhergehenden Ansprüche, **dadurch gekennzeichnet, dass** der Koppelmechanismus (60) als Scherenmechanismus ausgestaltet ist, und dass das erste Sperrglied (56) einem ersten Scherenhebel (62) und das zweite Sperrglied (58) einem zweiten Scherenhebel (64) zugeordnet ist.

5. Vorrichtung nach Anspruch 4, **dadurch gekennzeichnet, dass** der erste Scherenhebel (62) und der zweite Scherenhebel (64) gelenkig miteinander gekoppelt sind.

6. Vorrichtung nach Anspruch 4 oder 5, **dadurch gekennzeichnet, dass** das erste Sperrglied (56) und das zweite Sperrglied (58) relativ zueinander vorgespannt sind, um eine Relativverschwenkung zwischen dem ersten Sperrglied (56) und dem zweiten Sperrglied (58) zu bewirken.

7. Vorrichtung nach Anspruch 6, **dadurch gekennzeichnet, dass** der erste Scherenhebel (62) in Richtung auf einen ersten Anschlag (96) und der zweite Scherenhebel (64) in Richtung auf einen zweiten Anschlag (98) am Aktor (76), insbesondere an einem Anker (80) des Aktors (76), beaufschlagt ist.

8. Vorrichtung nach einem der vorhergehenden Ansprüche, **dadurch gekennzeichnet, dass** der Koppelmechanismus (60) als Kipphebelmechanismus gestaltet ist und einen Kipphebel (120) umfasst, der eine starre Verbindung zwischen dem ersten Sperrglied (56) und dem zweiten Sperrglied (58) aufweist und um eine Schwenkachse (124) verschwenkbar ist, um das erste Sperrglied (56) und das zweite Sperrglied (58) wechselseitig in die Sperrstellung zu überführen.

9. Vorrichtung nach Anspruch 8, **dadurch gekennzeichnet, dass** der Kipphebel (120) über ein Langloch (122) längsverschieblich und verschwenkbar mit der Schwenkachse (124) gekoppelt ist, und dass der Kipphebel (120) in Richtung auf das Formteil (18) vorgespannt ist.

10. Vorrichtung nach Anspruch 9, **dadurch gekennzeichnet, dass** der Kipphebel (120) über eine Längsverschiebung relativ zur Schwenkachse (124) bewegbar ist.

11. Vorrichtung nach einem der vorhergehenden Ansprüche, **gekennzeichnet durch** ein Rückstellelement (102), insbesondere ein Rückstellfeder, wobei das Rückstellelement (102) den Koppelmechanismus (60) in Richtung auf eine Ruhestellung beaufschlagt, wenn der Aktor (76) inaktiv ist.

12. Vorrichtung nach einem der vorhergehenden Ansprüche, **dadurch gekennzeichnet, dass** das Formteil (18) als Formschlauch auf Basis eines Silikonwerkstoffs oder eines Fluorkunststoffs gestaltet ist.

13. Vorrichtung nach einem der vorhergehenden Ansprüche, **gekennzeichnet durch** zumindest ein erstes Gehäuseteil (12) und zweites Gehäuseteil (14), die vorzugsweise über eine Schnappverbindung (22, 24) miteinander rastend verbindbar sind, wobei vorzugsweise der Aktor (76) am ersten Gehäuseteil (12) aufgenommen ist, und dass zumindest eines der Gehäuseteile (12, 14) eine definierte Aufnahmekontur (28) zur formschlüssigen Aufnahme des Formkörpers (18) aufweist.

14. Vorrichtung nach einem der vorhergehenden Ansprüche, **dadurch gekennzeichnet, dass** der Aktor (76) als elektromagnetischer Aktor (76) ausgebildet ist und ein C-förmiges oder U-förmiges Joch (78) ausweist, das mit einem Anker (80) gekoppelt ist, und dass das Joch (78) mit einer Mehrzahl von Spulen (82, 84, 86), vorzugsweise mit drei Spulen, gekoppelt ist, von denen jede einem Schenkel des Jochs (78) zugeordnet ist.

15. Verwendung eines Formteils (18) aus einem elastomeren Werkstoff als auswechselbares Einlegeteil in einer Vorrichtung zur Steuerung fluidischer Medien nach einem der Ansprüche 1 bis 14.

## Claims

1. Device for controlling fluid media in the form of a 3/2-way valve with three connections (40, 42, 44), comprising a molded part (18) made from an elastomeric material, two line sections (36, 38) which connect the three connections (40, 42, 44) to one another, between which two flow paths (46, 48) extend, and a control unit (16) comprising an electromagnetic actuator (76) that is coupled to two blocking elements (56, 58), wherein the two blocking elements (56, 58) are arranged to be displaced by the actuator (76) between a blocking position and a release position, wherein the actuator (76) is coupled to the two blocking elements (56, 58) to selectively block or enable a respective flow path (46; 48) of the two flow paths (46, 48), wherein a coupling mechanism (60) is provided, which is arranged to be driven by the actuator (76), wherein the coupling mechanism (60) mechanically couples the first blocking element (56) and the second blocking element (58) to each other, wherein the first blocking element (56) is in the blocking position when the second blocking element (58) is in the release position, and wherein the first blocking element (56) is in the release position when the second blocking element (58) is in the blocking position, **characterized in that** the first blocking element (56, 58) in the blocking position deforms a first line section (36, 38) to block the first flow path (46, 48), **in that** the second blocking element (56, 58) in the blocking position deforms a second line section (36, 38), in order to block the second flow path (46, 48), **in that** the two line sections (36, 38) are formed in the molded part (18), **in that** the molded part (18) has three connections (40, 42, 44) between which the two flow paths (46, 48) extend, **in that** the molded part (18) is arranged as an exchangeable insert on which grommets (52) are integrally formed, which are adjacent to the connections (40, 42, 44), and which ensure the alignment of the molded part (18) on the device, and **in that** the first blocking element (56) and the second blocking element (58) are coupled to each other in such a way that the first blocking element (56) and the second blocking element (58) simultaneously block the flow paths (46, 48) assigned thereto, at least in an intermediate position between the blocking position of the first blocking element (56) and the blocking position of the second blocking element (58).

2. The device according to claim 1, **characterized by** a biasing element (70, 134), which is coupled to the coupling mechanism (60) and acts on the first blocking element (56) and the second blocking element (58), at least in the intermediate position between the blocking position of the first blocking element (56) and the blocking position of the second blocking element (58) in the direction towards the line sections (36, 38) that are assigned thereto.

3. The device according to any one of the preceding claims, **characterized in that** at least one blocking element (56, 58) of the coupling mechanism (60) is coupled to a housing part (14) of the device via a living hinge (68).

4. The device according to any one of the preceding claims, **characterized in that** the coupling mechanism (60) is arranged as a scissor mechanism, and **in that** the first blocking element (56) is assigned to a first scissor lever (62) and the second blocking element (58) is assigned to a second scissor lever (64).

5. The device according to claim 4, **characterized in that** the first scissor lever (62) and the second scissor lever (64) are articulatedly coupled to each other.

6. The device according to claim 4 or 5, **characterized in that** the first blocking element (56) and the second blocking element (58) are biased relative to each other to cause relative pivoting between the first blocking element (56) and the second blocking element (58).

7. The device according to claim 6, **characterized in that** the first scissor lever (62) is biased towards a first stop (96) and the second scissor lever (64) is biased towards a second stop (98) at the actuator (76), in particular at an armature (80) of the actuator (76).

8. The device according to any one of the preceding claims, **characterized in that** the coupling mechanism (60) is arranged as a rocker arm mechanism and comprises a rocker arm (120), which has a rigid connection between the first blocking element (56) and the second blocking element (58), and which is pivotable about a pivot axis (124) in order to transfer the first blocking element (56) and the second blocking element (58) alternately into the blocking position.

9. The device according to claim 8, **characterized in that** the rocker lever (120) is coupled to the pivot axis (124) in a longitudinally displaceable and pivotable manner via an elongated hole (122), and **in that** the rocker lever (120) is biased towards the molded part (18).

10. The device according to claim 9, **characterized in that** the rocker lever (120) is movable by a longitudinal displacement in relation to the pivot axis (124).

11. The device according to any one of the preceding claims, **characterized by** a return element (102), in particular a return spring, wherein the return element (102) acts on the coupling mechanism (60) in the direction towards a rest position, when the actuator (76) is inactive.

12. The device according to any one of the preceding claims, **characterized in that** the molded part (18) is formed as a molded hose based on a silicone material or a fluoropolymer.

13. The device according to any one of the preceding claims, **characterized by** at least a first housing part (12) and a second housing part (14), which can be connected to one another in a latching manner, preferably via a snap connection (22, 24), wherein the actuator (76) is preferably accommodated on the first housing part (12), and in that at least one of the housing parts (12, 14) has a defined receiving contour (28) for a positive-fit support of the molded part (18).

14. The device according to any one of the preceding claims, **characterized in that** the actuator (76) is arranged as an electromagnetic actuator (76) and comprises a C-shaped or U-shaped yoke (78) that is coupled to an armature (80), and **in that** the yoke (78) is coupled to a plurality of coils (82, 84, 86), preferably three coils, each of which is associated with a leg of the yoke (78).

15. A use of a molded part (18) made from an elastomeric material as an exchangeable insert in a device for controlling fluid media according to any one of claims 1 to 14.

## Revendications

1. Dispositif de commande de milieux fluides sous la forme d'une vanne de distribution à 3/2 voies possédant trois raccords (40, 42, 44), comprenant une pièce moulée (18) en un matériau élastomère, deux portions de conduite (36, 38) qui relient ensemble les trois raccords (40, 42, 44) entre lesquels s'étendent deux chemins d'écoulement (46, 48), et comprenant une unité de commande (16) qui comporte un actionneur électromagnétique (76), lequel est accouplé avec deux organes de blocage (56, 58), les deux organes de blocage (56, 58) pouvant être déplacés par l'actionneur (76) entre une position de blocage et une position de libération, l'actionneur (76) étant accouplé avec les deux organes de blocage (56, 58) afin de bloquer ou de libérer sélectivement à chaque fois un chemin d'écoulement (46, 48) des deux chemins d'écoulement (46, 48), un mécanisme d'accouplement (60) étant présent, lequel peut être entraîné par l'actionneur (76), le mécanisme d'accouplement (60) accouplant mécaniquement l'un avec l'autre le premier organe de blocage (56) et le deuxième organe de blocage (58), le premier organe de blocage (56) se trouvant dans la position de blocage lorsque le deuxième organe de blocage (58) se trouve dans la position de libération et le premier organe de blocage (56) se trouvant dans la position de libération lorsque le deuxième organe de blocage (58) se trouve dans la position de blocage, **caractérisé en ce que** le premier organe de blocage (56, 58) dans la position de blocage déforme une première portion de conduite (36, 38) afin de bloquer le premier chemin d'écoulement (46, 48), **en ce que** le deuxième organe de blocage (56, 58) dans la position de blocage déforme une deuxième portion de conduite (36, 38) afin de bloquer le deuxième chemin d'écoulement (46, 48), **en ce que** les deux portions de conduite (36, 38) sont formées dans la pièce moulée (18), **en ce que** la pièce moulée (18) possède trois raccords (40, 42, 44) entre lesquels s'étendent les deux chemins d'écoulement (46, 48), **en ce que** la pièce moulée (18) est réalisée sous la forme d'une pièce d'insertion remplaçable, au niveau de laquelle sont façonnés des embouts (50), lesquels sont voisins des raccords (40, 42, 44) et garantissent l'orientation de la pièce moulée (18) au niveau du dispositif, et **en ce que** le premier organe de blocage (56) et le deuxième organe de blocage (58) sont accouplés l'un avec l'autre de telle sorte que le premier organe de blocage (56) et le deuxième organe de blocage (58), au moins dans une position intermédiaire entre la position de blocage du premier organe de blocage (56) et la position de blocage du deuxième organe de blocage (58), bloquent simultanément les chemins d'écoulement (46, 48) qui leur sont associés.

2. Dispositif selon la revendication 1, **caractérisé par** un élément de précontrainte (70, 134) qui est accouplé avec le mécanisme d'accouplement (60) et sollicite le premier organe de blocage (56) et le deuxième organe de blocage (58), au moins dans la position intermédiaire entre la position de blocage du premier organe de blocage (56) et la position de blocage du deuxième organe de blocage (58), en direction des portions de conduite (36, 38) qui leur sont associées.

3. Dispositif selon l'une des revendications précédentes, **caractérisé en ce qu'**au moins un organe de blocage (56, 58) du mécanisme d'accouplement (60) est accouplé par le biais d'une articulation de matériau (68) avec une partie de boîtier (14) du dispositif.

4. Dispositif selon l'une des revendications précédentes, **caractérisé en ce que** le mécanisme d'accouplement (60) est configuré sous la forme d'un mécanisme à pantographe et **en ce que** le premier organe de blocage (56) est associé à un premier levier croisé (62) et le deuxième organe de blocage (58) est associé à un deuxième levier croisé (64).

5. Dispositif selon la revendication 4, **caractérisé en ce que** le premier levier croisé (62) et le deuxième levier croisé (64) sont accouplés l'un avec l'autre de manière articulée.

6. Dispositif selon la revendication 4 ou 5, **caractérisé en ce que** le premier organe de blocage (56) et le deuxième organe de blocage (58) sont précontraints l'un par rapport à l'autre afin de produire un pivotement relatif entre le premier organe de blocage (56) et le deuxième organe de blocage (58).

7. Dispositif selon la revendication 6, **caractérisé en ce que** le premier levier croisé (62) est sollicité en direction d'une première butée (96) et le deuxième levier croisé (64) est sollicité en direction d'une deuxième butée (98) sur l'actionneur (76), notamment au niveau d'un élément d'ancrage (80) de l'actionneur (76).

8. Dispositif selon l'une des revendications précédentes, **caractérisé en ce que** le mécanisme d'accouplement (60) est configuré sous la forme d'un mécanisme à levier basculant et comporte un levier basculant (120) qui possède une liaison rigide entre le premier organe de blocage (56) et le deuxième organe de blocage (58) et peut pivoter autour d'un axe de pivotement (124) en vue de transférer le premier organe de blocage (56) et le deuxième organe de blocage (58) en alternance dans la position de blocage.

9. Dispositif selon la revendication 8, **caractérisé en ce que** le levier basculant (120) peut coulisser en longueur sur un trou oblong (122) et est accouplé de manière pivotante avec l'axe de pivotement (124), et **en ce que** le levier basculant (120) est précontraint en direction de la pièce moulée (18).

10. Dispositif selon la revendication 9, **caractérisé en ce que** le levier basculant (120) peut être déplacé par rapport à l'axe de pivotement (124) par le biais d'un coulissement en longueur.

11. Dispositif selon l'une des revendications précédentes, **caractérisé par** un élément de rappel (102), notamment un ressort de rappel, l'élément de rappel (102) sollicitant le mécanisme d'accouplement (60) en direction d'une position de repos lorsque l'actionneur (76) est inactif.

12. Dispositif selon l'une des revendications précédentes, **caractérisé en ce que** la pièce moulée (18) est configurée en tant que tuyau préformé à base d'un matériau en silicone ou d'une matière plastique fluorée.

13. Dispositif selon l'une des revendications précédentes, **caractérisé par** au moins une première partie de boîtier (12) et une deuxième partie de boîtier (14), lesquelles peuvent être assemblées par encliquetage l'une avec l'autre de préférence par le biais d'une liaison à encliquetage (22, 24), l'actionneur (76) étant de préférence accueilli au niveau de la première partie de boîtier (12), et en ce qu'au moins l'une des parties de boîtier (12, 14) possède un contour d'accueil (28) défini servant à l'accueil par complémentarité de formes du corps moulé (18).

14. Dispositif selon l'une des revendications précédentes, **caractérisé en ce que** l'actionneur (76) est réalisé sous la forme d'un actionneur électromagnétique (76) et possède une culasse (78) en forme de C ou en forme de U, laquelle est accouplée avec un induit (80), et **en ce que** la culasse (78) est accouplée avec une pluralité de bobines (82, 84, 86), de préférence avec trois bobines, dont chacune est associée à une branche de la culasse (78).

15. Utilisation d'une pièce moulée (18) en un matériau élastomère en tant que pièce d'insertion remplaçable dans un dispositif de commande de milieux fluides selon l'une quelconque des revendications 1 à 14.
